# EUROPEAN PATENT SPECIFICATION

(11) **EP 1 316 616 B1**
(45) Date of publication and mention of the grant of the patent: **28.01.2015**
(21) Application number: 02026225.9
(22) Date of filing: 26.11.2002
(51) Int. Cl.: C12P 19/34, C12Q 1/68, C12N 15/00

(54) **Method for producing linear DNA fragments for the in vitro expression of proteins**
Methode zur Herstellung linearer DNS Fragmente zur in vitro Proteinexpression
Méthode de production d'un fragment d'ADN linéaire pour l'expression in vitro de protéines

(30) Priority: 30.11.2001 DE 10158904
(43) Date of publication of application: 04.06.2003
(73) Proprietor: Roche Diagnostics GmbH, 68305 Mannheim (DE); F. Hoffmann-La Roche AG, 4070 Basel (CH)
(72) Inventor: JANY, Cordula, Dr., 85276 Pfaffenhofen/Ilm (DE); Buchberger, Bernd, Dr., 93197 Zeitlarn (DE); Watzele, Manfred, Dr., 82362 Weilheim (DE); Mutter, Wolfgang, Dr., 93047 Regensburg (DE); Roeder, Albert, Dr., 82541 Muensing (DE); Wessner, Stephanie, 82490 Farchant (DE)

(56) References cited:
- WO-A-00/34514
- WO-A-00/56914
- WO-A-98/11249
- US-A- 5 571 690
- US-B1- 6 232 107
- KAIN K C ET AL: "UNIVERSAL PROMOTER FOR GENE EXPRESSION WITHOUT CLONING: EXPRESSION-PCR" BIOTECHNIQUES, NATICK, MA, US, vol. 10, no. 3, March 1991 (1991-03), pages 366-368,370-372,374, XP000912135
- NAKANO HIDEO ET AL: "Efficient coupled transcription/translation from PCR template by a hollow-fiber membrane bioreactor." BIOTECHNOLOGY AND BIOENGINEERING, vol. 64, no. 2, 20 July 1999 (1999-07-20), pages 194-199, XP002237026 ISSN: 0006-3592
- SPIRIN A S: "CELL-FREE PROTEIN SYNTHESIS BIOREACTOR" AMERICAN CHEMICAL SOCIETY CONFERENCE PROCEEDINGS SERIES: FRONTIERS IN BIOPROCESSING, XX, XX, 1991, pages 31-43, XP001023126
- JACKSON M ET AL: "ENHANCED POLYPEPTIDE SYNTHESIS PROGRAMMED BY LINEAR DNA FRAGMENTS IN CELL-FREE EXTRACTS LACKING EXONUCLEASE V" FEBS LETTERS, XX, XX, vol. 163, no. 2, 14 November 1983 (1983-11-14), pages 221-224, XP008005956 ISSN: 0014-5793

### SEQUENCE LISTING

<110> Roche Diagnostics GmbH
<120> Method for producing linear DNA fragments for the in vitro expression of proteins
<130> 5711/00/
<160> 16
<170> PatentIn version 3.1
<210> 1
   <211> 30
   <212> DNA
   <213> Primer
<400> 1
   agaaggagat ataccatgac tagcaaagga 30
<210> 2
   <211> 30
   <212> DNA
   <213> Primer
<400> 2
   attcgccttt tattaatgat gatgatgatg 30
<210> 3
   <211> 41
   <212> DNA
   <213> Primer
<400> 3
   gaaattaata cgactcacta tagggagacc acaacggttt c 41
<210> 4
   <211> 37
   <212> DNA
   <213> Primer
<400> 4
   caaaaaaccc ctcaagaccc gtttagaggc cccaagg 37
<210> 5
   <211> 36
   <212> DNA
   <213> Primer
<400> 5
   actttaagaa ggagatatac catgactagc aaagga 36
<210> 6
   <211> 41
   <212> DNA
   <213> Primer
<400> 6
   gcgggtggct ccaagcgctc ccgggtttgt atagttcatc c 41
<210> 7
   <211> 32
   <212> DNA
   <213> Primer
<400> 7
   cgatcccgcg aaattaatac gactcactat ag 32
<210> 8
   <211> 31
   <212> DNA
   <213> Primer
<400> 8
   ctcctttcag caaaaaaccc ctcaagaccc g 31
<210> 9
   <211> 27
   <212> DNA
   <213> Primer
<400> 9
   gtagaggatc gagatctcga tcccgcg 27
<210> 10
   <211> 29
   <212> DNA
   <213> Primer
<400> 10
   gatatccgga tatagttcct cctttcagc 29
<210> 11
   <211> 31
   <212> DNA
   <213> Primer
<400> 11
   gatgccggcc acgatgcgtc cggcgtagag g 31
<210> 12
   <211> 30
   <212> DNA
   <213> Primer
<400> 12
   ggcgaccaca cccgtcctgt ggatatccgg 30
<210> 13
   <211> 54
   <212> DNA
   <213> Primer
<400> 13
   ggccgcttaa ttaaacatat gaccatcgaa ggccgcgccc caccacgcct catc 54
<210> 14
   <211> 44
   <212> DNA
   <213> Primer
<400> 14
   cggatcttac cggatcccgg gttatcatct gtcccctgtc ctgc 44
<210> 15
   <211> 41
   <212> DNA
   <213> Primer
<400> 15
   gaaattaata cgactcacta tagggagacc acaacggttt c 41
<210> 16
   <211> 37
   <212> DNA
   <213> Primer
<400> 16
   caaaaaaccc ctcaagaccc gtttagaggc cccaagg 37

## Description

The present disclosure concerns a method for producing linear DNA fragments for the *in vitro* expression of proteins in which a linear DNA fragment which contains control elements necessary for expression and the protein-coding gene which has complementary regions to the two ends of the linear DNA fragment at both ends, are amplified together using a primer pair which binds upstream and downstream of the expression control regions on the linear DNA fragment, characterized in that the linear DNA fragment can be produced by linearizing an expression vector. Furthermore a method is disclosed which concerns the preparation of the protein-coding gene the ends of which have regions which overlap with the linear DNA fragment. The present disclosure also encompasses the linear DNA fragments according to the present disclosure for the *in vitro* expression of proteins, the use of these fragments, kits containing the linear DNA fragments according to the present disclosure for the *in vitro* expression of proteins and methods for the *in vitro* expression of proteins starting from the linear DNA fragments according to the present disclosure.

Cell-free DNA-dependent *in vitro* transcription/translation based on E. coli lysates functions well in practice in conjunction with the expression of circular double helix DNA and in conjunction with the expression of long linear DNA. Attempts at expressing short linear pieces of DNA had only very limited success. The smaller the DNA used, the more difficult it is to obtain appreciable amounts of gene product. It was shown that these difficulties are partially due to the presence of exonucleases. Hence it was shown that exonuclease V is responsible for the degradation of linear DNA starting at the 3' end during *in vitro* transcription and translation using S30 lysates of *E. coli.*

mRNA degradation is often a control point for regulating gene expression in almost all organisms (Ross et al., 1995). Endoribonuclease E was identified as a key enzyme in the degradasome in *E. coli* (Grunberg-Manago et al., 1999). Invitrogen offers a BL21 strain which carries a mutation in the RnasE-coding gene and in which increased mRNA stability and increased protein yield are detectable. Prokaryotic mRNAs are also protected from exonucleolytic degradation by secondary structures at the 3' end (Klug et al., 1987; Mackie, 1987).

For the *in vitro* expression of linear DNA, long pieces of DNA offer the advantage of an increased protection of the DNA from exonucleases and an increased stability of the mRNA by the introduction of additional sequences.

A method for gene expression without cloning starting from PCR-generated DNAs was described for the first time in 1991 as so-called expression PCR (Kain et al., 1991). Expression PCR was used in eukaryotic (Kain & Lanar, 1994; Switzer & Heneine, 1995; Henkel & Beuerle, 1993; Resto et al., (1992) as well as in prokaroytic *in vitro* expression systems (Lesley et al., 1991; Martemyanov et al., 1997; Burks et al., 1997; Ohuchi et al., Nakano et al., 1999).

The following PCR techniques were used to generate linear DNAs:
WO00/34514 discloses a method in which a linear DNA fragment containing control elements and a protein coding gene are amplified together.

Hecht describes a method for the cell-free synthesis of a protein in US 5,571,690 starting from a template which was generated in a single step PCR reaction. In this process the entire gene sequence including the phage promoter region is amplified from one gene (see also Resto et al., 1992).

Martemyatov et al., 1997 demonstrate the synthesis of a protein in a cell-free system based on *E. coli* S30 extracts starting from a template which was produced in a 2-step PCR reaction. In this process the target gene was amplified with the aid of two gene-specific primers. The T7 promoter and the ribosomal binding site was subsequently fused on in a second PCR reaction using so-called megaprimers (163 nucleotides long). The incorporation of promoters and ribosomal binding sites by means of PCR primers is also described in current books on PCR methods (PCR by Newton & Graham, 1994, Spectrum publisher, Heidelberg; PCR McPherson and Moller, 2000, BIOS Scientific Publishers, Oxford).

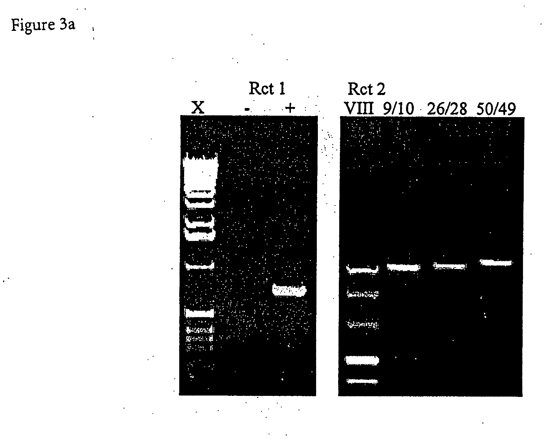

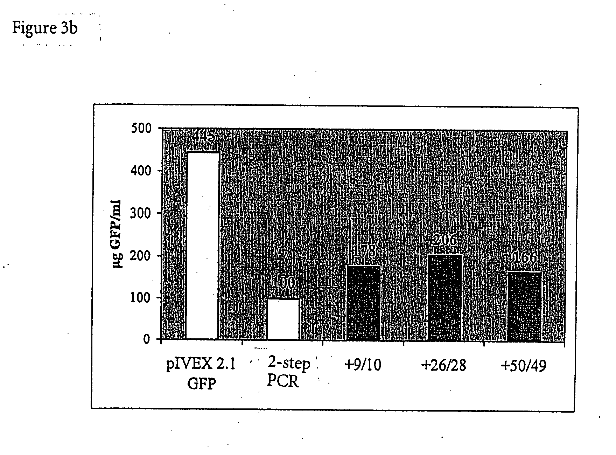

Ohuchi et al., 1998 use the method that was also used by Kain et al., 1991 of overlap extension PCR to produce linear DNA templates. This PCR method was first described by Horton et al. 1989 as gene splicing by overlap extension (SOE) for the fusion of genes without restriction enzymes by means of overlapping PCR fragments. A primer pair is used in a first PCR reaction for the expression PCR by SOE which each carries a homologous region to the DNA segments to be fused at the 5' end. DNA pieces to be fused on are then present in the second PCR reaction (cf. Kain et al., 1991, figure 3).

In their Patent Application WO 00/56914 Liang and Felgner describe a method for producing transcriptionally active DNA molecules based on an SOE-PCR in which a DNA fragment with a promoter, a DNA fragment with a terminator, a primer pair which is homologous to the 5' ends of these DNA fragments and a primer pair that is homologous to the 3' end of the DNA fragments and to the 5' end of the gene sequence to be expressed were amplified together.

The production of DNA templates for cell-free protein synthesis by means of a one step PCR offers no advantage since all genes to be amplified have to be present beforehand in a corresponding expression plasmid. A disadvantage of two step PCR is the synthesis of the outer, extremely long primers which is ineffective and costly. The protein expression yield of a two step PCR using long external primers is very low due to the low stability of the PCR product and of the mRNA transcribed therefrom. Furthermore the PCR reaction itself causes more problems the longer the primers since the probability of dimer formation increases. For expression PCR by means of SOE the DNA molecules to be fused on have to be firstly prepared and laboriously eluted through a gel (cf. Ohuchi et al., 1998).

The object of the present invention was therefore to provide a simple method for producing linear DNA fragments for the *in vitro* expression of proteins. The aim was to avoid complicated cloning steps. Moreover the DNA fragments should have an increased protection against exonucleases and an increased stability of the mRNA. As a result the linear DNA fragments should enable high protein yields in cell-free *in vitro* transcription/translation systems.

The object was achieved according to the invention by a method for expressing a protein in a cell-free expression system using a lysate from E. *coli,* comprising the steps: (a) linearizing in the multiple cloning site an expression vector which contains the T7 promoter, a ribosomal binding site and the T7 terminator as control elements, (b) amplifying by PCR the linearized vector fragment of step (a) together with the protein-coding gene whose two ends have complementary regions to the two ends of the linearized vector, using a primer pair which binds upstream of the T7 promoter and downstream of the T7 terminator on the vector, wherein the protein-coding gene whose ends have regions that are complementary to the two ends of the linearized vector fragment is produced in a PCR reaction in which the protein-coding gene is amplified using gene-specific primers whose 5' end have regions which overlap the linearized vector fragment and in this manner the amplified gene is extended by the overlapping regions of the primers, wherein a linear DNA fragment for the *in vitro* expression of the protein is produced; and (c) *in vitro* transcription and translation, characterized in that the regions of the amplified gene which overlap the linearized vector fragment have a length from 21 to 22 nucleotides.

Herein is further disclosed a method for producing linear DNA fragments for the *in vitro* expression of proteins in which a linear DNA fragment which contains control elements necessary for expression is amplified together with the protein-coding gene whose two ends have complementary regions to the two ends of the linear DNA fragment, using a primer pair which binds upstream and downstream of the expression control regions on the linear DNA fragment, characterized in that the linear DNA fragment can be produced by linearizing an expression vector.

The expression vector can be linearized by simple restriction digestion in the multiple cloning site, by double restriction digestion in the multiple cloning site or by cutting out a gene cloned into the expression vector. Another method for producing a vector fragment is amplification by means of inverse PCR.

Suitable expression vectors are those which provide all regulatory elements required by the corresponding expression system. In the case of prokaryotic *in vitro* expression systems based on lysates from *E. coli* and the T7 system, all expression vectors coding for a T7 promoter, a ribosomal binding site and a T7 terminator are suitable such as e.g. pIVEX vectors, all T7 expression vectors of the pET family, pCRT7-TOPO vectors, pDEST vectors, pCAL vectors. In native *E. coli* expression systems without T7 polymerase, all vectors are suitable which code for an endogenous *E. coli* promoter, a ribosomal binding site and a terminator such as pHB6, pVB6, pXB, pBH, pBV, pBX, pASKIBA vectors, pBAD and pTrxFus. In eukaryotic expression systems all expression vectors are suitable which are functional in the corresponding in vivo system. Preferred expression vectors are those in which the sequences and distances of the regulatory elements are optimized for protein expression e.g. pIVEX vectors for the prokaryotic *in vitro* expression system based on lysates of *E. coli* and the T7 system.

Due to the inventive solution of preparing the linear DNA fragment which contains the control elements required for protein expression by linearizing an expression vector, the linear DNA fragment already contains all necessary control elements for the *in vitro* expression of proteins. Furthermore there is an ideal spacing between the regulatory elements themselves and between the regulatory elements and the gene to be expressed. For example the distance between the stop and terminator in the *E. coli* system plays a decisive role with regard to the expression rate. Another advantage of the method is that in addition to the control regions it is also readily possible to fuse various tags and long fusion partners, protease recognition sequences and/or CAP structures for eukaryotic ribosomes by means of amplification. The present disclosure also encompasses systems in which the linearized vector has been further cleaved or where several linearized vectors are used. The control elements required for expression can also be divided among two vectors which are then linearized.

However, it is also possible to use fragmented linearized vectors for the *in vitro* expression of proteins. In this variant of the present disclosure the linear DNA fragment which contains the control elements necessary for expression is fragmented i.e. cleaved. The cleaved fragments that are obtained are then amplified according to the present disclosure together with the protein coding gene whose two ends contain complementary regions to the two DNA fragments containing control elements using a primer pair which binds upstream and downstream. The following example is intended to illustrate this variant of the present disclosure in more detail. The linearized vector "V" has a T7 promoter, a ribosomal binding site and a T7 terminator before cleavage. Fragments "V1" and "V2" are then prepared of which "V1" has no T7 promoter and "V2" no longer has a T7 terminator. The inventive method is now carried out as described above using "V1" and "V2" instead of "V"; i.e. "V1" and "V2" are amplified together with the protein-coding gene which has a complementary region to "V1" at one end and a complementary region to "V2" at the other end, the primer pair binding correspondingly upstream and downstream on "V1" and "V2".

Another advantage of the method according to the present disclosure is that the gene sequence to be expressed is fused into the vector in the first PCR cycles with the linear vector which codes for a resistance gene. Since the vector arms serve as primers in the first cycles, the complete plasmid is cosynthesized to a certain extent. This has the advantage that the final product can not only be used for expression in cell-free systems but also directly for transformation in a suitable bacterial strain. Hence the PCR product is secured in the plasmid as a bacterial clone.

The protein-coding gene whose ends have a complementary region to the ends of the linear DNA fragment can be produced according to the present disclosure in a PCR reaction in which the protein-coding gene is amplified using gene-specific primers whose 5' ends have regions which overlap with the ends of the linear DNA fragment and hence the amplified gene is extended by the overlapping regions of the primers. For this purpose the gene-specific sense primer has at least five additional nucleotides at the 5' end which are identical to the 3' end of the upper strand of the linear DNA fragment, and the gene-specific antisense primer has at least five additional nucleotides at the 5' end which are identical to the 3' end of the lower strand of the linear DNA fragment.

The process steps of extending the protein-coding gene and amplifying this gene together with the linear DNA fragment which contains the control elements required for protein expression such as a promoter and terminator region can be carried out in a common reaction as well as in successive reactions.

For the *in vitro* expression of proteins using lysates of *E. coli* the linear vector preferably contains the T7 promoter, the ribosomal binding site and the T7 terminator.

Furthermore it is preferred according to the present disclosure that the linearized vector codes for a C-terminal or N-terminal tag sequence or for a fusion protein sequence which is then automatically introduced at the same time as the control elements required for expression.

The amplification can be carried out by PCR protocols known to a person skilled in the art (PCR by Newton & Graham, 1994, publisher Spektrum, Heidelberg; PCR by McPherson and Moller, 2000, BIOS Scientific Publishers Oxford).

The present disclosure concerns in particular a kit for producing linear DNA fragments for the *in vitro* expression of proteins which is present in one or several containers comprising:
- a linear DNA fragment which can be produced by linearizing an expression vector and contains all control elements required for the expression of proteins.
- outer primers which bind upstream and downstream of the expression control region on the linear DNA fragment. The outer primer pair can also bind directly to the 5' end of the promoter and terminator region.

The kit according to the present disclosure can additionally contain, in the same or separate containers, specific primers whose 5' ends have regions which overlap the ends of the linear DNA fragment. For this purpose the gene-specific sense primer carries at least five additional nucleotides at its 5' end which are identical to the 3' end of the upper strand of the linear DNA fragment and the gene-specific antisense primer carries at least five additional nucleotides at its 5' end which are identical to the 3' end of the lower strand of the linear DNA fragment.

The kit according to the present disclosure may additionally contain DNA polymerases with or without proof-reading activity or a mixture of the two as well as the required buffer and magnesium chloride solutions and deoxynucleotide triphosphates, and reagents for optimizing PCR reactions such as DMSO, glycerol, formamide, betaine, 7-deaza GTP etc.. In another preferred embodiment of the kit the linear DNA fragments contain C-terminal or N-terminal tag sequences or a fusion protein sequence such as the His tag, HA tag and GFP sequence.

The present disclosure concerns the use of the kit according to the present disclosure to produce a linear DNA fragment for the *in vitro* expression of proteins.

A further subject matter of the present disclosure is a linear DNA fragment which can be obtained by the inventive method described above where the PCR fragment contains a protein-coding gene and the control elements required for protein expression such as a promoter and terminator region.

For the *in vitro* expression of proteins using lysates from *E. coli,* the linear DNA fragment preferably contains the T7 promoter, the ribosomal binding site and the T7 terminator.

In a another preferred embodiment the linear DNA fragment contains a C-terminal or N-terminal tag sequence or a fusion protein sequence such as the His tag, the HA tag and the GFP sequence.

The present disclosure also concerns the use of the linear DNA fragment according to the disclosure for the *in vitro* expression of a protein in a cell-free expression system. In particular the present disclosure concerns the use of the linear DNA fragment according to the disclosure for the *in vitro* expression of a protein using a lysate from bacterial strains or eukaryotic cells.

Another subject matter of the present disclosure is a method for expressing proteins comprising the steps:
- producing a linear DNA fragment by the inventive method described above,
- in vitro transcription and/or translation.

The *in vitro* expression of the protein is preferably carried out in a cell-free expression system. Such *in vitro* translation methods have been described for various prokaryotic and eukaryotic systems (Nirenberg and Matthaei, 1961; Zubay 1973; Jackson and Hunt, 1983; Erickson and Blobel, 1983; Matthews and Colman 1991).

It is particularly preferred that the *in vitro* expression of the protein is carried out in a coupled system using a lysate from bacterial strains or eukaryotic cells (Zubay 1973; Baranov et al., 1989; Kigawa and Yokoyama, 1991; Kudlicki et al., 1992; Spirin, 1992; Baranov and Spirin, 1993). The *in vitro* expression of proteins can also be carried out in a CFCF or CECF reactor (Spirin et al., 1988).

In particular it is preferred that the *in vitro* expression of the protein is carried out using a lysate from *E. coli*. Furthermore it is preferable that the method according to the present disclosure for the *in vitro* expression of a protein is characterized in that the protein-coding gene is amplified directly from a gene bank or RNA fraction by PCR or RT-PCR.

### Figure legends

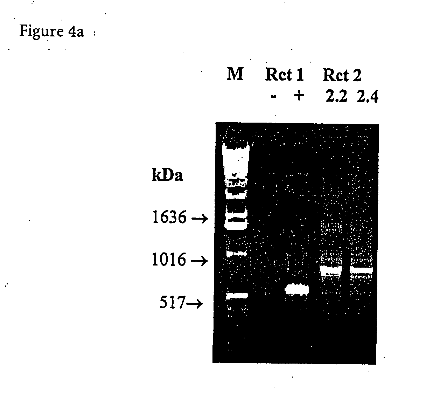

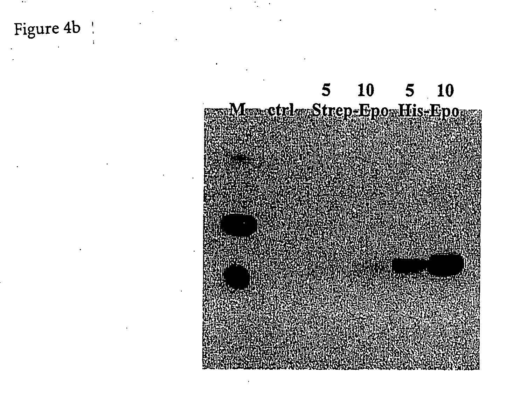

**Figure 1**
   Method for producing linear DNA fragments for the *in vitro* expression of proteins
   Complementary regions are attached to the 5' ends of the gene to be expressed by means of primers in a first gene-specific PCR reaction. A linearized expression vector is present in the second PCR whose ends can hybridize with the first PCR product by means of the attached complementary ends. In several intermediary steps the 3' ends of the two vector strands serve as primers and are extended after hybridization with the first PCR product. The 3' ends of the first PCR product are also extended after hybridization with the vector ends. Subsequently the PCR product to be expressed is produced in several amplification cycles using a second primer pair which bind upstream and downstream of the promoter and terminator (in this example the T7 system).
**Figure 2a**
   - lane M: DNA molecular weight marker
   - lane 1: 5 µl GFP PCR product from the first gene-specific PCR
   - lane 2: negative control of the overlap extension PCR without the first gene product
   - lane 3: 5 µl product from the overlap extension PCR (GFP-His)
**Figure 2b**
   The amount of active GFP was measured in a fluorimeter after expression in the RTS100 E. coli HY kit. A comparison was made between the plasmid pIVEX2.1GFP, a PCR product produced using long outer primers (2-step PCR GFP) and two different volumes of the product produced by the overlap extension PCR.
**Figure 3a**
   - lane X: DNA molecular weight marker
   - lane -: negative control of the gene-specific PCR without template
   - lane +: 5 µl product from the first gene-specific first PCR (GFP)
   - lane VII: DNA molecular weight marker
   - lane 9/10: 3 µl GFP PCR product from the overlap extension PCR (GFP Strep tag) using the primer combination 9/10 (SEQ ID NOs.: 7 and 8)
   - lane 26/28: 3 µl GFP PCR product from the overlap extension PCR (GFP Strep tag) using the primer combination 26/28 (SEQ ID NOs.: 9 and 10)
   - lane 50/49: 3 µl GFP PCR product from the overlap extension PCR (GFP Strep tag) using the primer combination 50/49 (SEQ ID NOs.: 11 and 12)
**Figure 3b**
   The amount of active GFP was measured in a fluorimeter after expression in the RTS100 E. coli HY kit. A comparison was made between the plasmid pIVEX2.1GFP, a GFP-PCR product produced using long outer primers (2-step PCR) and the three different overlap extension PCR products prepared using outer primer pairs.
**Figure 4a**
   - lane M: DNA molecular weight marker
   - lane -: negative control of the gene-specific PCR without template
   - lane +: 5 µl product from the first gene-specific first PCR (Epo)
   - lane 2.2: 5 µl Epo PCR product from the overlap extension PCR (Epo Strep tag)
   - lane 2.4: 5 µl Epo PCR product from the overlap extension PCR (Epo His tag).
**Figure 4b**
   The amount of Epo with a Strep tag and a His tag after expression in the RTS100 E. coli HY kit was analysed by a Western blot. 2 µl of each of the linear Epo expression constructs shown in figure 4a (lane 2.2 and 2.4) were expressed in the RTS100. 5 and 10 µl of these expression solutions were separated in a 4-12 % Nu PAGE gel and subsequently blotted on a membrane. The detection was by means of an anti-Epo MAB. M = protein molecular weight marker; ctrl = negative control of the overlap extension PCR after expression in the RTS100.

### Examples

### Example 1

### Overlap extension PCR using a linearized pIVEX2.3 plasmid and GFP expression data

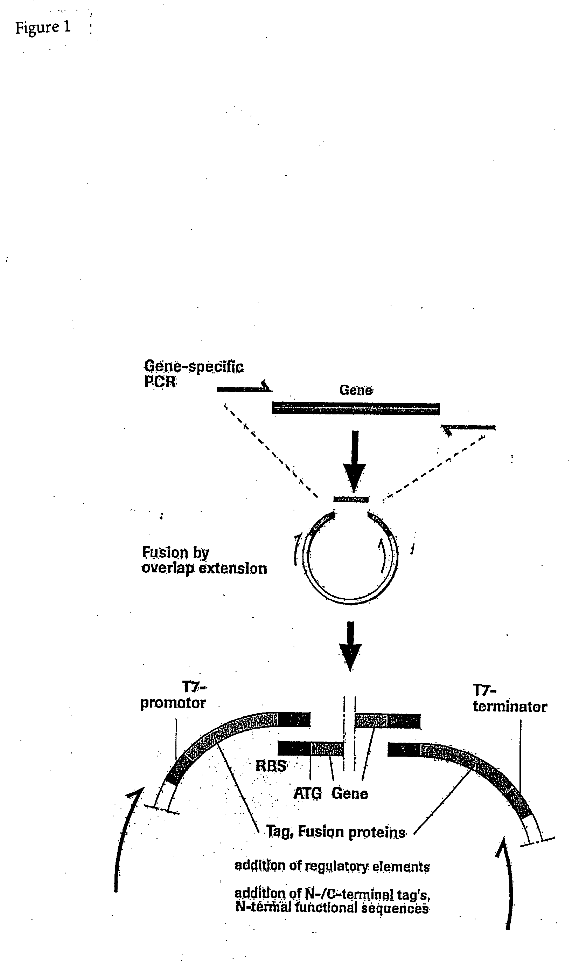

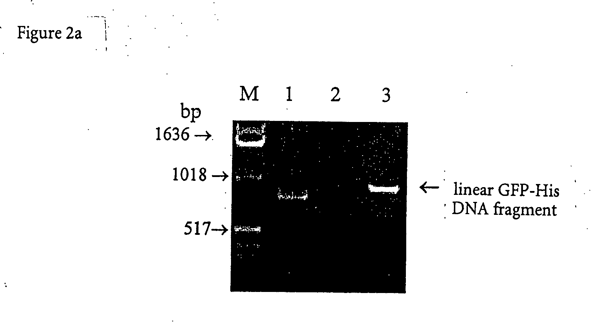

The first PCR reaction (5 µl applied in lane 1, figure 2a) was carried out under the following conditions in a 50 µl mixture: 5 µl 10 x PCR buffer containing 1.5 mM MgCl₂ final concentration, 0.5 µM primer A, 0.5 µM primer B, 250 µM dNTP's (Roche), 3 U Pwo (Roche). 100 ng of the plasmid pCRIITOPO-GFP which carries the sequence for GFP (green fluorescent protein) with a C-terminal His tag sequence without regulatory expression elements was used as the DNA template. The following oligonucleotide was used as the sense primer A:
A GFP 15/15 SEQ ID No.: 1: 5'-AGA AGG AGA TAT ACC ATG ACT AGC AAA GGA-3' and the following was used as the antisense primer B:
   B GFPmw SEQ ID No.: 2: 5'-ATT CGC CTT TTA TTA ATG ATG ATG ATG ATG-3'. The program 94° 4'-20 x (94° 1'-50° 1'- 72° 1')-4° was run on a Gene Amp 9600 PCR instrument from Perkin Elmer.

The second PCR reaction (5 µl applied in lane 3 figure 2a; lane 2 in figure 2a shows the second PCR control reaction without the first PCR product) was carried out under the following conditions in a 50 µl mixture: 5 µl 10 x PCR buffer containing 1.5 mM MgCl₂ final concentration, 0.5 µM sense primer, 0.5 µM antisense primer, 250 µM dNTP's (Roche), 3 U Pwo (Roche). 2 µl of the first PCR product was added as the template. In addition 10 ng of plasmid pIVEX2.3 which had been linearized with NcoI/SmaI and subsequently eluted from the agarose gel was present. The following primers were used: Sense primer Md0 SS:
SEQ ID No.: 3:
   5'-GAA ATT AAT ACG ACT CAC TAT AGG GAG ACC ACA ACG GTT TC-3'
      and antisense primer Md14 Ter AS
SEQ ID No.: 4:
   5'-CAA AAA ACC CCT CAA GAC CCG TTT AGA GGC CCC AAG G-3'

The program 94° 4'-30 x (94° 1'-60° 1'- 72° 1'30")-4° was run on a Gene Amp 9600 PCR instrument from Perkin Elmer.

The GFP expression construct (figure 2a, lane 3) prepared by the overlap extension PCR method using linearized pIVEX2.3 plasmid was incubated for 16 hours at 30°C and 650 rpm in 50 µl mixtures according to the instructions of the Rapid Translation System RTS 100 *E. coli* HY kit. 2.2 and 4.4 µl of the product shown in figure 2a, lane 3 were used, 0.5 µg of the plasmid DNA pIVEX2.1-GFP was used and compared with the same amounts of the GFP PCR product which was prepared by a 2-step PCR reaction using long primers without an overlap extension PCR with the linearized pIVEX plasmid.

The incorporation of promoters and ribosomal binding sites by means of long PCR primers is described in current books on PCR methods (PCR by Newton & Graham, 1994, Publisher Spektrum, Heidelberg; PCR McPherson and Moller, 2000, BIOS Scientific Publishers, Oxford). All products were measured in a fluorimeter.

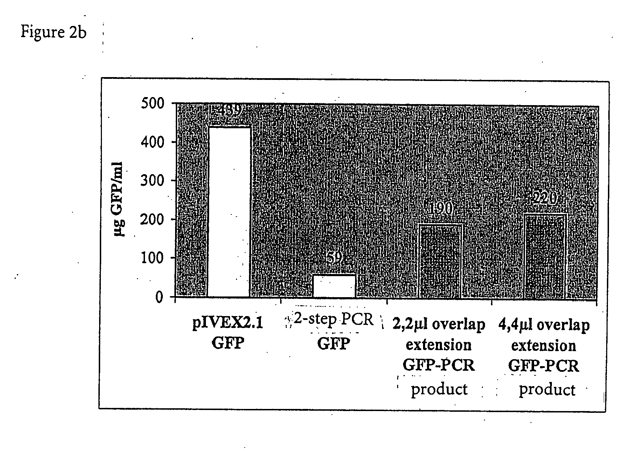

Only a small amount of additional non-translated sequence can be introduced in the 2-step PCR reaction using long primers (problems occur in the long primer synthesis and PCR reaction) and hence the DNA product and the transcribed mRNA are unstable and yield small amounts of protein. Protein yields that were more than twice as high were achieved by the overlap extension PCR method using a linearized plasmid which results in a larger stable DNA fragment (figures 2a/b).

### Example 2

### GFP expression data with the overlap extension PCR product produced using a linearized pIVEX2.1 plasmid and various second primer pairs

The first PCR reaction (Rct 1 +, 5 µl applied; figure 3a) was carried out under the following conditions in a 50 µl mixture: 5 µl 10 x Pwo buffer without MgSO₄, 6 µl 25 mM MgSO₄ (final concentration 3 mM), 1.5 mM MgCl₂ final concentration, 0.5 µM primer A, 0.5 µM primer B, 250 µM dNTP's (Roche), 3 U Pwo (Roche). 500 ng of the plasmid pCRIITOPO-2.3 GFP which contains the sequence for GFP (green fluorescent protein) with a C-terminal His tag sequence without regulatory expression elements was used as the DNA template. The following oligonucleotide was used as the sense primer (A): A GFP 21/15
SEQ ID No.: 5
5'-ACT TTA AGA AGG AGA TAT ACC ATG ACT AGC AAA GGA-3'
and the following oligonucleotide was used as the antisense primer (B):
B GFP 22/18 2.1 over
SEQ ID No.: 6:
5'-GCG GGT GGC TCC AAG CGC TCC CGG GTT TGT ATA GTT CAT CC-3'

The program 94° 4'-20 x (94° 1'-50° 1'- 72° 1')-4° was run on a Gene Amp 9600 PCR instrument from Perkin Elmer.

4 different outer primer pairs were used for the second PCR reactions which bound at different distances upstream and downstream of the T7 promoter and T7 terminator on the added linearized pIVEX2.1 plasmid. The second PCR reactions (Rct 2; 3 µl were applied each time, figure 3a) were carried out in 50 µl mixtures under the following conditions: 5 µl 10 x Pwo buffer without MgSO₄, 6 µl 25 mM MgSO₄ (final concentration 3 mM), 0.5 µM sense primer, 0.5 µM antisense primer, 250 µM dNTP's (Roche), 3 U Pwo (Roche). 2 µl of the first PCR product was added as the template. In addition 10 ng of the plasmid pIVEX2.1 that had been linearized with NcoI/SmaI and subsequently eluted from the agarose gel were present.

The following primer pairs were used:
pair 9/10 sense primer M + 9 SS
SEQ ID No.: 7:
   5'-CGA TCC CGC GAA ATT AAT ACG ACT CAC TAT AG-3'
and antisense primer M + 10 AS
SEQ ID No.: 8:
   5'-CTC CTT TCA GCA AAA AAC CCC TCA AGA CCC G-3'.

   This primer pair hybridizes 9 nucleotides upstream of the T7 promoter and 10 nucleotides downstream of the T7 terminator.
pair 26/28 sense primer M + 26 SS
SEQ ID No.: 9:
   5'-GTA GAG GAT CGA GAT CTC GAT CCC GCG-3'
and antisense primer M + 28 AS
SEQ ID No.: 10:
   5'-GAT ATC CGG ATA TAG TTC CTC CTT TCA GC-3'

   This primer pair hybridizes 26 nucleotides upstream of the T7 promoter and 28 nucleotides downstream of the T7 terminator.
pair 50/49 sense primer M + 50 SS
SEQ ID No.: 11:
   5'-GAT GCC GGC CAC GAT GCG TCC GGC GTA GAG G-3'
and antisense primer M + 49 AS
SEQ ID No.: 12:
   5'-GGC GAC CAC ACC CGT CCT GTG GAT ATC CGG-3'.

This primer pair hybridizes 50 nucleotides upstream of the T7 promoter and 49 nucleotides downstream of the T7 terminator.

The program 94° 4'-30 x (94° 1'-60° 1'- 72° 1'30")-4° was run on a Gene Amp 9600 PCR instrument from Perkin Elmer.

The GFP expression constructs of various lengths (figure 3a, Rct 2) prepared by the overlap extension PCR method using linearized pIVEX2.1 plasmid were incubated for 4 hours at 30°C and 650 rpm in 50 µl mixtures according to the instructions of the Rapid Translation System RTS 100 *E. coli* HY kit. 200 ng of each of the second PCR products and 500 ng pIVEX2.1-GFP were used per 50 µl mixture and compared (2 step PCR) with the same amounts of the GFP PCR product which was prepared by a 2-step PCR reaction using long primers without an overlap extension PCR with the linearized pIVEX plasmid. All products were measured in a fluorimeter.

The GFP expression constructs of various lengths produced by the overlap extension PCR using a linearized pIVEX2.1 plasmid exhibited a considerably increased expression rate compared with the 2-step GFP-PCR product. In contrast to example 1 the regions which overlapped the linearized vector were in this case only 21 and 22 nucleotides long. This overlapping region was sufficient for a positive overlap extension PCR reaction. The extension of the second PCR products was clearly demonstrated using the second primer pairs which hybridized at different distances upstream and downstream of the T7 promoter and T7 terminator sequence. Using GFP as the test gene, a distance of ca. 30 bp on the other side of the T7 regulatory element was found to be ideal.

### Example 3

### Addition of Strep and His tag sequences and of a protease recognition sequence to the sequence for human erythropoietin by means of overlap extension PCR

The first erythropoietin (Epo)-specific PCR reaction (Rct 1 +; 5 µl applied, figure 4a) was carried out under the following conditions in a 50 µl mixture: 5 µl 10 x Pwo buffer without MgSO₄, 6 µl 25 mM MgSO₄ (final concentration 3 mM), 0.5 µM Epo-specific sense primer, 0.5 µM Epo-specific antisense primer, 250 µM dNTP's (Roche), 3 U Pwo (Roche). 300 ng of the plasmid pcDNA3-Epo from Dr. Johannes Auer which carried the sequence for human erythropoietin without a Tag sequence was used as the DNA template. The following oligonucleotide was used as the sense primer:
2X Xa Epo
SEQ ID No.: 13:
   5'-GGC CGC TTA ATT AAA CAT ATG ACC ATC GAA GGC CGC GCC CCA CCA CGC CTC ATC-3'
and the following oligonucleotide was used as the antisense primer: 3'EPO/Vec correct
SEQ ID No.: 14:
   5'-CGG ATC TTA CCG GAT CCC GGG TTA TCA TCT GTC CCC TGT CCT GC-3'

The program 94° 4'-20 x (94° 1'-50° 1'- 72° 1')-4° was run on a Gene Amp 9600 PCR instrument from Perkin Elmer.

The second PCR reactions (5 µl each) (figure 4a) were carried out under the following conditions in a 50 µl mixture: 5 µl 10 x Pwo buffer without MgSO₄, 6 µl 25 mM MgSO₄ (final concentration 3mM), 0.5 µM sense primer, 0.5 µM antisense primer, 250 µM dNTP's (Roche), 3 U Pwo (Roche). 2 µl of the first PCR product was added as the template. In addition 10 ng of plasmid pIVEX2.2 which had been linearized with NdeI/SalI and subsequently eluted from agarose gel which codes for the Strep tag or 10 ng of plasmid pIVEX2.4 linearized with NdeI/SalI and subsequently eluted from agarose gel which codes for the His tag were present.

The following primers were used:
sense primer Md0 SS
SEQ ID No.: 15:
   5'-GAA ATT AAT ACG ACT CAC TAT AGG GAG ACC ACA ACG GTT TC-3'
and antisense primer Md14 Ter AS
SEQ ID No.: 16
5'-CAA AAA ACC CCT CAA GAC CCG TTT AGA GGC CCC AAG G-3'

The program 94° 4'-30 x (94° 1'-60° 1'- 72° 2')-4° was run on a Gene Amp 9600 PCR instrument from Perkin Elmer.

The Epo expression constructs (figure 4a) prepared by the overlap extension PCR method using linearized pIVEX2.2 or pIVEX2.4 plasmid were incubated for 4 hours at 30°C and 650 rpm in 50 µl mixtures according to the instructions of the Rapid Translation System RTS 100 *E. coli* HY kit. 5 µl and 10 µl aliquots of the products from the second PCR reaction were used for expression. 2 µl of each of the expression mixtures was admixed with sample buffer (Novex), heated for 5 min to 95°C, cooled on ice and separated on a 4-12 % NuPAGE gel (Novex) containing 1 x MES buffer at 200 V. The proteins were transferred onto a nitrocellulose membrane (Protan, Schleicher & Schuell) by 30 min electroblotting at 30 V. The membrane was blocked for 1 hour with 3 % BSA solution in a single concentrated TBS-T buffer, washed with single concentrated TBS-T buffer and incubated for 1 hour with MAB anti-Epo-POD-coupled antibody (Roche) in single concentrated TBS-T. After washing three times with single concentrated TBS-T, the proteins were detected by means of Lumi Light plus Western blotting substrate (Roche) according to the manufacturer's instructions.

The initial construct pcDNA Epo coded for neither of the two tags and could also not be expressed in a prokaryotic *in vitro* system since it was a eukaryotic expression plasmid. Hence this showed that the Strep tag and the His tag including the protease recognition sequences (in this case the sequence for factor Xa protease) and the regulatory elements required for expression could be introduced by means of overlap extension PCR and that Epo could be successfully expressed.

## Claims

1. Method for expressing a protein in a cell-free expression system using a lysate from *E. coli,* comprising the steps:
(a) linearizing in the multiple cloning site an expression vector which contains the T7 promoter, a ribosomal binding site and the T7 terminator as control elements,
(b) amplifying by PCR the linearized vector fragment of step (a) together with the protein-coding gene whose two ends have complementary regions to the two ends of the linearized vector, using a primer pair which binds upstream of the T7 promoter and downstream of the T7 terminator on the vector, wherein
the protein-coding gene whose ends have regions that are complementary to the two ends of the linearized vector fragment is produced in a PCR reaction in which the protein-coding gene is amplified using gene-specific primers whose 5' end have regions which overlap the linearized vector fragment and in this manner the amplified gene is extended by the overlapping regions of the primers, wherein a linear DNA fragment for the *in vitro* expression of the protein is produced; and
(c) *in vitro* transcription and translation,
**characterized in that**
the regions of the amplified gene which overlap the linearized vector fragment have a length from 21 to 22 nucleotides.

## Patentansprüche

1. Verfahren zum Exprimieren eines Proteins in einem zellfreien Expressionssystem unter Verwendung eines Lysats von E. *coli,* umfassend die folgenden Schritte:
(a) Linearisieren eines Expressionsvektors, der den T7-Promoter, eine Ribosomenbindungsstelle und den T7-Terminator als Kontrollelemente enthält, in der multiplen Klonierungsstelle,
(b) Amplifizieren des linearisierten Vektorfragments von Schritt (a) zusammen mit dem proteincodierenden Gen, dessen beide Enden zu den beiden Enden des linearisierten Vektors komplementäre Regionen aufweisen, mittels PCR, unter Verwendung eines Primerpaars, das stromaufwärts des T7-Promoters und stromabwärts des T7-Terminators auf dem Vektor bindet, wobei die proteincodierenden Gene, deren Enden Regionen, die zu den beiden Enden des linearisierten Vektorfragments komplementär sind, aufweisen, in einer PCR-Reaktion erzeugt wird, in der das proteincodierende Gen unter Verwendung von genspezifischen Primern amplifiziert wird, deren 5'-Ende Regionen, die mit dem linearisierten Vektorfragment überlappen, aufweist, und das amplifizierte Gen dadurch um die überlappenden Regionen der Primer verlängert wird, wobei ein lineares DNA-Fragment für die in-vitro-Expression des Proteins erzeugt wird; und
(c) in-vitro-Transkription und -Translation,
**dadurch gekennzeichnet, dass**
die Regionen des amplifizierten Gens, die mit dem linearisierten Vektorfragment überlappen, eine Länge von 21 bis 22 Nukleotiden aufweisen.

## Revendications

1. Procédé d'expression d'une protéine dans un système d'expression acellulaire au moyen d'un lysat d'E. *coli,* comprenant les étapes :
(a) linéarisation dans le site de clonage multiple d'un vecteur d'expression qui contient le promoteur T7, un site de liaison ribosomique et le terminateur T7 comme éléments de contrôle,
(b) amplification par PCR du fragment de vecteur linéarisé de l'étape (a) avec le gène codant pour la protéine dont les deux extrémités ont des régions complémentaires des deux extrémités du vecteur linéarisé, au moyen d'une paire d'amorces qui se lie en amont du promoteur T7 et en aval du terminateur T7 sur le vecteur, dans lequel
le gène codant pour la protéine dont les extrémités ont des régions qui sont complémentaires des deux extrémités du fragment de vecteur linéarisé est produit dans une réaction de PCR dans laquelle le gène codant pour la protéine est amplifié au moyen d'amorces spécifiques du gène dont l'extrémité 5' a des régions qui chevauchent le fragment de vecteur linéarisé et de cette manière le gène amplifié est prolongé par les régions de chevauchement des amorces, dans lequel un fragment d'ADN linéaire pour l'expression *in vitro* de la protéine est produit ; et
(c) transcription et traduction *in vitro,*
**caractérisé en ce que**
les régions du gène amplifié qui chevauchent le fragment de vecteur linéarisé ont une longueur de 21 à 22 nucléotides.
